(19) Europäisches Patentamt
European Patent Office
Office européen des brevets

(11) Publication number: 0 219 271 B1

(12) EUROPEAN PATENT SPECIFICATION

(45) Date of publication of patent specification: 23.05.90

(51) Int. Cl.[5]: C 07 C 4/06, C 07 C 11/02 // B01J29/28

(21) Application number: 86307555.2

(22) Date of filing: 01.10.86

# (54) Catalytic conversion of propane to ethylene over ZSM-50.

(30) Priority: 07.10.85 US 784967

(43) Date of publication of application: 22.04.87 Bulletin 87/17

(45) Publication of the grant of the patent: 23.05.90 Bulletin 90/21

(84) Designated Contracting States: BE DE FR GB IT NL

(56) References cited:
EP-A-0 170 486
GB-A-1 563 345
US-A-4 547 618

(73) Proprietor: MOBIL OIL CORPORATION
150 East 42nd Street
New York New York 10017 (US)

(72) Inventor: Kaeding, Warren William
6 Roseberry Court
Lawrenceville New Jersey 08648 (US)

(74) Representative: Cooper, John Anthony et al
Mobil Court 3 Clements Inn
London WC2A 2EB (GB)

EP 0 219 271 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Ethylene is prepared commercially by heating ethane, propane, higher paraffins or naphtha, diluted with steam, at about 850°C, 1550°F, for very short contact times, without a catalyst. Highest ultimate yields come from ethane (81%), propane (43%) and n-butane (41.5%). All world-scale plants with billion-pound-per-year ethylene capacity are based on this thermal cracking/dehydrogenation technology. Although a host of rival schemes has been studied, none have reached commercial application.

Weaknesses in the established process are (1) high reaction temperature and low hydrocarbon partial pressure, (2) low product separation/purification temperatures −100 to −130°C (−150 to −200°F) and high pressure 3500 kPa (500 psig), (3) relatively low yields from $C_3$ and higher feeds, (4) a complex mixture of products, and (5) relatively high capital and operating costs.

Olefins have been prepared from methanol over ZSM—5 with low activity, $SiO_2/Al_2O_3$ 300/1, M. M. Wu and W. W. Kaeding, J. Cat. *88* 478 (1984). In the major $C_2$—$C_4$ olefins product, ethylene is usually the smallest component (10—15 wt%). When n-butane was used with these same catalysts, propylene and $C_5$+ olefins were produced with only traces of ethylene. When propane is converted over catalysts with various oxides on silica or alumina such as chromium oxide, propylene is the major product.

Accordingly, the present invention provides a process for converting propane to ethylene by contact with a zeolite catalyst characterized by using ZSM—50 as the catalyst.

ZSM—50 is disclosed in EPA 170486.

ZSM—50 is characterized by a distinctive X-ray diffraction pattern substantially as shown in Table 1.

Table 1

| Interplanar d-Spacing (A) | Relative Intensity, $I/I_o$ |
|---|---|
| 20.1 ± .3 | W |
| 11.1 ± .17 | S |
| 10.1 ± .16 | M |
| 9.7 ± .14 | W |
| 5.77 ± .09 | W |
| 5.61 ± .09 | W |
| 4.64 ± .07 | M |
| 4.35 ± .07 | M |
| 4.30 ± .07 | VS |
| 4.00 ± .06 | S |
| 3.85 ± .06 | M |
| 3.70 ± .06 | M |
| 3.42 ± .05 | W |
| 3.35 ± .05 | W |
| 3.27 ± .05 | M |
| 3.24 ± .05 | W |
| 2.94 ± .04 | W |
| 2.53 ± .04 | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the spectrometer. From these, the relative intensities, 100 $I/I_o$, where $I_o$ is the intensity of the strongest line or peak, and d (obs.) the interplanar spacing

in Angstrom Units (A), corresponding to the recorded lines, were determined. In Table 1, the relative intensities are given in terms of the symbols W=weak, M=medium, S=strong and VS=very strong. In terms of intensities, these may be generally designated as follows:

W=0—20
M=20—40
S=40—60
VS=60—100

ZSM—50 with a low silica to alumina ratio has a formula, on an anhydrous basis and in terms of moles or oxides per 100 moles of silica, as follows:

$$(0—4)R_2O:()—10)M_{2/n}O:(1—5)Al_2O_3:(100)SiO_2$$

wherein M is an alkali or alkaline earth metal, n is the valence of M, and R is an organic cation of diquaternary directing agent compound generally expressed by the following formula:

$$X(CH_3)_3N(CH_2)_6N(CH_3)_3X$$

wherein X is an anion, e.g. halide, such as iodide.

Such ZSM—50 can be prepared from a reaction mixture containing sources of an alkali or alkaline earth metal oxide, an oxide of aluminum, an oxide of silicon, an organic cation and water and having a composition, in terms of mole ratios of oxides, within the following ranges:

| Reactants | Useful | Preferred |
|---|---|---|
| $SiO_2/Al_2O_3$ | 20—100 | 30—90 |
| $OH^-/SiO_2$ | 0.1—0.6 | 0.1—0.3 |
| $R/SiO_2$ | 0.05—0.6 | 0.1—0.3 |
| $M/SiO_2$ | 0.01—1.0 | 0.1—0.6 |

wherein M is an alkali or alkaline earth metal and R is an organic cation derived from the above identified diquaternary directing agent compound.

Crystallization of such ZSM—50 can be carried out at either static or stirred condition in a suitable reactor vessel, such as for example, polypropylene jars or teflon lined or stainless steel authclaves. The total useful range of temperatures for crystallization is usually 100°C to 200°C for 48 hours to 15 days. Thereafter, the crystals are separated from the liquid and recovered.

This invention may be performed with either high or low silica to alumina ratio ZSM—50.

In accordance with the present invention, the zeolite catalyst may be contacted with anhydrous acidic oxide gas capable of accepting hydrogen by reacting therewith. Examples of such gases include oxidative dehydrogenation agents such as sulfur dioxide ($SO_2$) and nitrous oxide ($N_2O$). Such oxide gases may be contacted with the zeolites by a pretreatment procedure, e.g., prior to any catalytic use, or as a cofeed with the propane reactant.

The zeolites suitable for use in accordance with the present invention may be combined with another material resistant to the temperatures and other conditions employed in the present organic conversion process. Such matrix materials include active and inactive materials and synthetic or naturally occurring zeolites as well as inorganic materials such as clays, silica and/or metal oxides, e.g. alumina. The latter may be either naturally occurring or in the form of gelatinous precipitates, sols or gels including mixtures of silica and metal oxides. Use of a material in conjunction with the zeolite, i.e. combined therewith, which is active, may enhance the conversion and/or selectivity of the catalyst in certain organic conversion processes. Inactive materials suitably serve as diluents to control the amount of conversion in a given process so that products can be obtained economically and orderly without employing other means for controlling the rate or reaction. Frequently, crystalline silicate materials have been incorporated into naturally occurring clays, e.g. bentonite and kaolin. These materials, i.e. clays, oxides, etc., function, in part, as binders for the catalyst. It is desirable to provide a catalyst having good crush strength, because in use the catalyst may be subjected to rough handling, which tends to break the catalyst down into powder-like materials which cause problems in processing.

Naturally occurring clays which can be composited with the zeolite include the montmorillonite and kaolin families which include the subbentonites, and the kaolins commonly known as Dixie, McNamee, Georgia and Florida clays, or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolite catalyst hereby synthesized can be composited with a porous matrix material such as silica-alumina, silica-magnesia, silica-zirconia, silica-thoria, silica-beryllia, silica-titania, as well as ternary compositions such as silica-alumina-thoria, silica-alumina-zirconia, silica-alumina-magnesia and silica-magnesia-zirconia. The matrix can be in the form of a cogel. A mixture of these components could also be used. The relative proportions of finely divided crystalline silicate and

matrix vary widely with the crystalline silicate content ranging from about 1 to about 90 percent by weight, and more usually in the range of about 2 to about 50 percent by weight of the composite.

Conditions for converting propane in accordance with the present invention may include a temperature of 100°C to 700°C, a pressure of 0.1 atmosphere to 60 atmospheres, and a weight hourly space velocity of 0.5 to 400. The feedstock, in addition to propane, may optionally comprise, e.g., up to about 98% of a diluent gas, especially an inert diluent gas. The feedstock may also comprise a small percentage, e.g., 1 percent by weight or less, of impurities associated with propane feedstocks such as butane.

Example 1

High silica ZSM—50 sample was synthesized with dibenzyldimethylammonium chloride as directing agent. The product was filtered, water-washed and dried at 120°C. X-ray diffraction shows a highly crystalline ZSM—50. Compositional data are, wt%:

| | | |
|---|---|---|
| $SiO_2$ | 80.4 | |
| $Al_2O_3$ | 0.65 | |
| N | 0.63 | |
| Na | 0.57 | |
| Ash | 86.16 | |
| $SiO_2/Al_2O_3$ | 210 | molar ratio |

Low silica ZSM—50 was prepared with Diquat-6 bromide $[Br(CH_3)_3N(CH_2)_6N(CH_3)_3Br]$ as directing agent.

The mixture was crystallized, with stirring, at 160°C for 4 days. The product was 90% crystalline ZSM—50. Compositional data are, wt%:

| | | |
|---|---|---|
| $SiO_2$ | 83.0 | |
| $Al_2O_3$ | 3.6 | |
| N | 1.29 | |
| Na | 0.27 | |
| Ash | 86.38 | |
| $SiO_2/Al_2O_3$ | 39.2 | molar ratio |

Both preparations were converted to the ammonium form by a preliminary calcination at 500°C in $N_2$ followed by air. They were then treated with 10% $NH_4Cl$ solution.

In Examples which follow, ZSM—50 samples of Example 1 were used to convert propane. Reagent grade propane containing 0.9% n-butane was used without further purification. Corrections were made for the butane in runs with low conversion. Five to ten grams of catalyst wafers, crushed and screened to 0.8 to 1.2 mm (14—20 mesh), were used in glass screening reactors. Effluent gas was sampled in a hot syringe and analyzed for hydrocarbons. A second sample was analyzed with an argon carrier gas to measure hydrogen. Material balances of ±5% were usually obtained.

When sulfur dioxide was used, it was used in accordance with the following procedure:

A. Sulfur dioxide, 20 cc/min, was passed over the catalyst for 30—60 min at 300°C, followed by calcination in air for 30—60 min at 500°C.

B. After treatment of the catalyst as described in A, above, 1—3 wt% $SO_2$ was added to the propane feed for the screening reaction.

Screening tests at various temperatures (400—650-C) and weight hourly space velocities (WHSV) ranging from 0.86 to 6.9 were used with propane. In addition, sulfur dioxide (1—5%) was used to modify the catalyst and/or as a hydrogen acceptor. Ideally, it would aid dehydrogenation of propane, Eq. 1, by consuming hydrogen thereby preventing the reverse reaction, Eq. 2. Higher conversions to propylene should occur. The well-known propane cracking raction, Eq. 3, occurs and is interesting because the desired ethylene is a product. A catalyst and hydrogen acceptor that would reduce methane formation and increase ethylene yield, Eq. 4, is a desirable objective.

Dehydrogenation

$$CH_3CH_2CH_3 \rightleftarrows CH_3CH{=}CH_2 + H_2 \qquad (1)$$

$$3CH_3CH_2CH_3 + SO_2 \longrightarrow 3CH_3CH{=}CH_2 + H_2S + SO_2 \qquad (2)$$

Cracking

$$CH_3CH_2CH_3 \longrightarrow CH_2{=}CH_2 + CH_4 \qquad (3)$$

Objective

$$3CH_3CH_2CH_3 + SO_2 \longrightarrow 3CH_3{=}CH_2 + CH_3CH{=}CH_2 + 2H_2O + H_2S \qquad (4)$$

Example 2

The HZSM—50 sample having a $SiO_2/Al_2O_3$ molar ratio of 200/1 was used to convert propane.

Screening results for propane are summarized in Table 2. Conversion increased significantly with increases in temperature and contact time. The highest ethylene selectivity observed was 35%, Run 7. Significant amounts of propylene (15—20% selectivity) and methane (22—29%) were also obtained.

Catalytic amounts (1%) of sulfur dioxide and nitrous oxide were added to the propane feed streams to determine whether olefin yield could be increased. Results are summarized in Table 3. In almost every case, a small increase in ethylene selectivity was observed.

Table 2
Conversion of Propane over HZSM-50, $SiO_2/Al_2O_3$=210/1

| Run No. | 1 | 2 | 3 | 4 | 5 | 6 | 7 | 8 | 9 |
|---|---|---|---|---|---|---|---|---|---|
| Temp. °C | 550 | 550 | 550 | 600 | 600 | 600 | 650 | 650 | 650 |
| WHSV $C_3H_8$ | 3.46 | 1.73 | .864 | 3.46 | 1.73 | .864 | 3.46 | 1.73 | .864 |
| Conversion | 6 | 10 | 14 | 19 | 28 | 40 | 41 | 53 | 73 |
| Selectivity, wt. % | | | | | | | | | |
| BTX(a) | 0 | .6 | 2.8 | 3.1 | 5.0 | 10.5 | 5.6 | 11.8 | 15.2 |
| $C_9$+ | 0 | 9.0 | 1.0 | 5.1 | 1.7 | 3.7 | .9 | 2.6 | 4.8 |
| Tot. Liq. Prod. | 0 | 9.6 | 3.8 | 8.2 | 6.7 | 14.2 | 6.5 | 14.4 | 20.0 |
| $H_2$ | .7 | .5 | .6 | .9 | 1.0 | 1.0 | 1.4 | 1.5 | 1.9 |
| $CO/CO_2$ | 0 | 0 | 0 | 0 | 0 | 0 | 0 | 0 | 0 |
| $CH_4$ | 22.3 | 22.6 | 25.6 | 24.2 | 26.1 | 27.0 | 26.6 | 26.4 | 29.5 |
| $C_2H_6$ | 2.8 | 5.1 | 7.8 | 4.4 | 6.5 | 8.4 | 5.3 | 6.8 | 8.9 |
| $C_2H_4$ | 31.8 | 28.2 | 24.1 | 32.3 | 28.6 | 21.0 | 34.6 | 27.0 | 21.3 |
| $C_3H_8$ | SM(b) | SM | SM | SM | SM | SM | SM | SM | SM |
| $C_3H_6$ | 17.5 | 17.1 | 18.1 | 19.9 | 19.1 | 16.0 | 20.5 | 16.8 | 12.8 |
| $C_4H_{10}$ | 24.9 | 12.3 | 12.8 | 5.2 | 5.0 | 5.6 | .6 | 1.5 | 1.2 |
| $C_4H_8$ | 0 | 4.6 | 7.2 | 4.9 | 7.0 | 6.8 | 4.5 | 5.6 | 4.4 |
| $C_2$-$C_4$ | 27.7 | 17.4 | 20.6 | 9.6 | 11.5 | 14.0 | 5.9 | 8.3 | 10.1 |
| | 49.3 | 49.9 | 49.4 | 57.1 | 54.7 | 43.8 | 59.6 | 49.4 | 38.5 |
| Total | 100.0 | 100.0 | 100.0 | 100.0 | 100.0 | 100.0 | 100.0 | 100.0 | 100.0 |

(a) Benzene, toluene, xylene.
(b) SM = starting material.

Table 3
Conversion of Propane over HZSM-50, $SiO_2/Al_2O_3$=210/1 with $SO_2$

| Run No. | 10 | 11 | 12 | 13 | 14 | 15 |
|---|---|---|---|---|---|---|
| Temp. °C | 600 | 600 | 600 | 650 | 650 | 650 |
| WHSV $C_3H_8$ | 3.46 | 1.73 | .864 | 3.64 | 1.73 | .864 |
| $SO_2$ | .005 | .005 | .005 | .005 | .005 | .005 |
| Conversion | 20 | 26 | 41 | 42 | 57 | 73 |
| Selectivity, wt. % | | | | | | |
| BTX | 1.5 | 4.3 | 11.2 | 4.4 | 10.9 | 17.7 |
| $C_9$+ | 1.5 | 1.1 | 6.3 | .4 | 3.5 | 5.9 |
| Tot. Liq. Prod. | 3.0 | 5.4 | 17.5 | 4.8 | 14.4 | 23.6 |
| $H_2$ | .7 | .8 | 1.0 | 1.1 | 1.2 | 1.5 |
| $CO/CO_2$ | 0 | .2 | .3 | 0 | .1 | .2 |
| $CH_4$ | 26.4 | 26.7 | 26.0 | 27.2 | 26.8 | 27.5 |
| $C_2H_6$ | 4.4 | 6.5 | 8.6 | 5.1 | 6.7 | 8.9 |
| $C_2H_4$ | 35.4 | 29.7 | 20.2 | 34.5 | 27.4 | 20.7 |
| $C_3H_8$ | SM | SM | SM | SM | SM | SM |
| $C_3H_6$ | 19.8 | 19.2 | 15.3 | 20.1 | 16.8 | 12.6 |
| $C_4H_{10}$ | 5.8 | 5.2 | 5.5 | 1.5 | 1.3 | 1.1 |
| $C_4H_8$ | 4.5 | 6.3 | 5.6 | 5.7 | 5.4 | 3.9 |
| Total | 100.0 | 100.0 | 100.0 | 100.0 | 100.0 | 100.0 |

Example 3

In a manner similar to Example 2, the more active catalyst of Example 1, containing higher concentrations of aluminum ($SiO_2/Al_2O_3=39$) was tested. Results are summarized in Table 4. In comparison with the previous run ($SiO_2/Al_2O_3=210$), modest increases in conversion were observed. However, the amount of ethylene and total $C_2$—$C_4$ olefins was relatively low. By doubling the space velocity, Runs 5—8, Table 4, olefine selectivity increased at the expanse of lower conversion.

The amount of sulfur dioxide in the feed stream was increased about 20 fold, compared with Example 2, to determine whether it was reacting with propane to remove hydrogen, Eqs. 2 and 4. Significant and encouraging increases in ethylene and propylene selectivities were observed, Table 5, Runs 12, 16. Some elemental sulfur appeared to be present in the product. It was removed by passing the gas stream through alumina at low temperature.

Table 4
HZSM-50, $SiO_2/Al_2O_3$ = 39/1
Propane

| Run No. | 1 | 2 | 3 | 4 | 5 | 6 | 7 | 8 |
|---|---|---|---|---|---|---|---|---|
| Temp. °C | 500 | 550 | 600 | 650 | 500 | 550 | 600 | 650 |
| WHSV $C_3H_8$ | 3.5 | 3.5 | 3.5 | 3.5 | 6.9 | 6.9 | 6.9 | 6.9 |
| Conversion | 15 | 28 | 46 | 47 | 9 | 20 | 35 | 31 |
| Selectivity, wt. % | | | | | | | | |
| BTX | 4.1 | 10.2 | 11.8 | 13.8 | 2.7 | 6.7 | 11.4 | 10.0 |
| $C_9$+ | 1.0 | 2.3 | 3.1 | 2.8 | 11.0 | 2.5 | 2.0 | 2.3 |
| Tot. Liq. Prod. | 5.1 | 12.5 | 14.9 | 16.6 | 13.7 | 9.2 | 13.4 | 12.3 |
| $H_2$ | .6 | 1.1 | 1.8 | 2.3 | .6 | 1.1 | 1.6 | 1.9 |
| $CO/CO_2$ | 0 | 0 | 0 | 0 | 0 | 0 | 0 | 0 |
| $CH_4$ | 15.8 | 23.1 | 28.2 | 26.0 | 14.2 | 21.9 | 25.0 | 24.2 |
| $C_2H_6$ | 8.9 | 12.2 | 11.7 | 7.7 | 5.4 | 8.7 | 7.9 | 5.6 |
| $C_2H_4$ | 8.5 | 12.8 | 18.2 | 23.4 | 12.6 | 18.3 | 23.2 | 27.8 |
| $C_3H_8$ | SM | SM | SM | SM | SM | SM | SM | SM |
| $C_3H_6$ | 9.5 | 12.1 | 13.6 | 16.8 | 13.8 | 16.5 | 17.6 | 21.1 |
| $C_4H_{10}$ | 44.6 | 20.7 | 6.3 | 3.0 | 36.0 | 18.1 | 5.5 | 2.4 |
| $C_4H_8$ | 7.0 | 5.5 | 5.3 | 4.2 | 3.7 | 6.2 | 5.8 | 4.7 |
| Total | 100.0 | 100.0 | 100.0 | 100.0 | 100.0 | 100.0 | 100.0 | 100.0 |

Table 5
HZSM-50, $SiO_2/Al_2O_3$ = 39/1
Propane

| Run No. | 9 | 10 | 11 | 12 | 13 | 14 | 15 | 16 |
|---|---|---|---|---|---|---|---|---|
| Temp. °C | 500 | 550 | 600 | 650 | 500 | 550 | 600 | 650 |
| WHSV $C_3H_8$ | 6.91 | 6.91 | 6.91 | 6.91 | 3.46 | 3.46 | 3.46 | 3.46 |
| $SO_2$ | .105 | .105 | .105 | .105 | .105 | .105 | .105 | .105 |
| Conversion | 10 | 13 | 18 | 29 | 10 | 14 | 28 | 50 |
| Selectivity, wt. % | | | | | | | | |
| BTX | 5.7 | 3.9 | 3.2 | 3.1 | 5.7 | 4.8 | 5.8 | 8.1 |
| $C_9+$ | 4.4 | 2.7 | 1.5 | .7 | 3.2 | 1.4 | 1.8 | 1.7 |
| Tot. Liq. Prod. | 10.1 | 6.6 | 4.7 | 3.8 | 8.9 | 6.2 | 7.6 | 9.8 |
| $H_2$ | .3 | .5 | .7 | 1.0 | .4 | .6 | .9 | 1.6 |
| $CO/CO_2$ | 1.3 | .9 | .6 | .4 | .7 | .7 | .4 | .4 |
| $CH_4$ | 11.7 | 17.0 | 21.7 | 24.3 | 13.9 | 22.1 | 25.0 | 26.7 |
| $C_2H_6$ | 3.6 | 4.5 | 5.3 | 4.9 | 6.7 | 8.9 | 8.2 | 6.4 |
| $C_2H_4$ | 14.7 | 21.9 | 28.0 | 33.1 | 11.4 | 22.4 | 26.8 | 31.1 |
| $C_3H_8$ | SM | SM | SM | SM | SM | SM | SM | SM |
| $C_3H_6$ | 14.9 | 18.9 | 21.1 | 22.5 | 11.9 | 10.1 | 19.1 | 18.6 |
| $C_4H_{10}$ | 43.4 | 26.1 | 13.4 | 5.3 | 43.0 | 16.0 | 6.5 | 1.3 |
| $C_4H_8$ | 0 | 3.6 | 4.5 | 4.7 | 3.1 | 5.0 | 5.5 | 4.1 |
| Total | 100.0 | 100.0 | 100.0 | 100.0 | 100.0 | 100.0 | 100.0 | 100.0 |

## Claims

1. A process for converting propane to ethylene by contact with a zeolite catalyst characterized by using ZSM—50 as the catalyst.

2. The process of claim 1 further characterized in that the ZSM—50 is in a binder.

3. The process of claim 1 further characterized in that the ZSM—50 contacts an anhydrous acidic oxide gas capable of accepting hydrogen by reacting therewith prior to contact with propane.

4. The process of any preceding claim wherein an anhydrous acid oxide gas capable of accepting hydrogen by reacting therewith is cofed with the propane.

5. The process of claim 3 or 4 wherein the acidic oxide gas is sulfur dioxide.

## Patentansprüche

1. Verfahren zur Umwandlung von Propan in Ethylen durch Kontakt mit einem Zeolithkatalysator, gekennzeichnet durch die Verwendung von ZSM—50 als Katalysator.

2. Verfahren nach Anspruch 1, weiterhin dadurch gekennzeichnet, daß ZSM—50 in einem Bindemittel vorliegt.

3. Verfahren nach Anspruch 1, weiterhin dadurch gekennzeichnet, daß ZSM—50 vor dem Kontakt mit Propan mit einem wasserfreien sauren Oxidgas in Kontakt gebracht wird, das durch Reaktion Wasserstoff aufnehmen kann.

4. Verfahren nach einem der vorstehenden Ansprüche, worin das wasserfreie saure Oxidgas, das durch Reaktion Wasserstoff aufnehmen kann, gleichzeitig mit dem Propan zugeführt wird.

5. Verfahren nach Anspruch 3 oder 4, worin das saure Oxidgas Schwefeldioxid ist.

## Revendications

1. Une procédé pour convertir du propane en éthylène par contact avec un catalyseur de type zéolite, caractérisé en ce qu'une ZSM—50 est utilisée comme catalyseur.

2. Une procédé suivant la revendication 1, caractérisé en ce que la ZSM—50 est dans un liant.

3. Une procédé suivant la revendication 1, caractérisé en ce que la ZSM—50 est mise en contact avec un gaz anhydre de type oxyde acide capable d'accepter l'hydrogène par réaction avec celui-ci, avant d'être mise en contact avec le propane.

4. Une procédé suivant l'une quelconque des revendications précédentes, caractérisé en ce que le gaz anhydre de type oxyde acide capable d'accepter l'hydrogène par réaction avec celui-ci, est co-alimenté avec le propane.

5. Une procédé suivant la revendication 3 ou 4, caractérisé en ce que le gas de type oxyde acide est du dioxyde de soufre.